# EUROPEAN PATENT APPLICATION

(11) **EP 4 751 721 A1**
(43) Date of publication of application: **03.06.2026**
(21) Application number: 23946765.7
(22) Date of filing: 24.07.2023
(51) Int. Cl.: A61K 31/37, A61P 25/16, A61P 25/28, A23L 33/10

(54) **COMPOSITION CONTAINING NOVEL COMPOUND FOR PREVENTING OR TREATING PARKINSON'S DISEASE**

(71) Applicant: PRG S&Tech Inc., Busan 46274 (KR)
(72) Inventor: PARK, Bum-Joon, Busan 46241 (KR)
(74) Representative: Mewburn Ellis LLP
(86) International application number: PCT/KR2023/010642
(87) International publication number: WO 2025/023339

(57) **Abstract**

The present invention relates to a use of a novel compound for preventing, alleviating, or treating Parkinson's disease, the novel compound exhibiting an effect of inhibiting synuclein aggregation in a Parkinson's disease (PD) mouse model. As a result of histological analysis, it was confirmed that the loss of dopaminergic neurons was blocked by treatment with the novel compound. Therefore, the novel compound can be effectively utilized in the development of a therapeutic agent for Parkinson's disease.

## Description

### Technical Field

The present disclosure relates to a composition for preventing or treating Parkinson's disease, including a novel compound.

### Background Art

Parkinson's disease is a progressive neurodegenerative disease that increases with age and is caused by a decrease in dopamine neurons present in the midbrain dopamine pathway responsible for motor function. Thus, the main symptoms include movement disorders such as muscle stiffness and tremors of the limbs.

The exact cause of Parkinson's disease is unknown, but it is believed to be related to environmental factors such as neurotoxins such as pesticides, genetic factors, mitochondrial dysfunction, and aging. Genetic factors are known to be associated with mutations in genes such as alpha-synuclein, Parkin, PINK-1, UCH-L1, and DJ-1. Currently, there are many medications that can alleviate the symptoms of Parkinson's disease, but no medication has yet been reported that is able to stop the progression of the disease, and chronic use of medications carries a high risk of causing side effects that weaken the mind and body.

Known medications for treating Parkinson's disease include L-dopa preparations, dopamine receptor agonists, anticholinergic drugs, and Eldepryl. However, most of these drugs do not treat the cases but rather serve to manage symptoms, requiring continuous medication. Long-term administration of these drugs causes side effects. For example, anticholinergic drugs may cause autonomic nervous system disorders or mental dysfunction, which limits their continuous administration to elderly patients. Additionally, in the case of L-dopa preparations, the effectiveness gradually decreases with long-term intake, and side effects occur, such as body twisting or abnormal movements of the hands and feet. In addition, although surgical treatments such as high-frequency neurostimulation, high-frequency ablation, or deep brain stimulation are also being performed, they require invasive surgery and are expensive.

Therefore, since the exact cause of Parkinson's disease is not known, treatment methods are involved mainly to alleviate symptoms rather than to treat the root cause, such that there is an urgent need to develop new, more effective treatments for the prevention and treatment of Parkinson's disease.

### Disclosure of the Invention

### Technical Goals

To this end, an object of the present disclosure is to provide a use for preventing, ameliorating, or treating Parkinson's disease, containing a novel compound.

### Technical Solutions

To achieve the above object, the present disclosure provides a pharmaceutical composition for preventing or treating Parkinson's disease, including a compound represented by the following Chemical Formula 1 or a pharmaceutically acceptable salt thereof as an active ingredient: 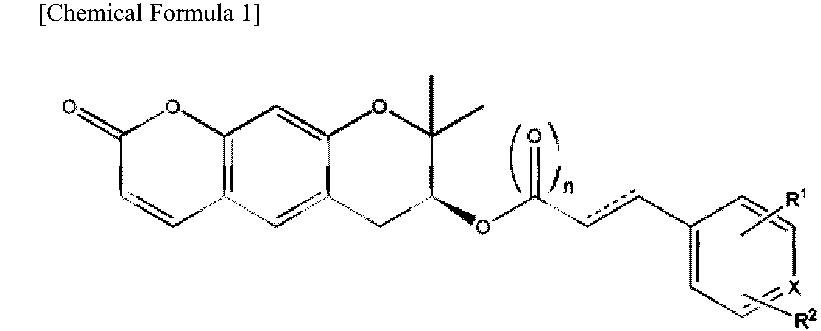 wherein, in the Chemical Formula 1,
- - - - - - is a single bond or a double bond,
n is an integer between 0 and 1,
X is CH or N, and
R¹ and R² may be the same or different and each independently selected from hydrogen, (C1~C4)alkyl, (C1~C4)alkoxy, hydroxy, halo, nitro, cyano, or (C1∼C4)alkylcarboxy.

In addition, the present disclosure provides a health functional food composition for preventing or ameliorating Parkinson's disease, including a compound represented by the Chemical Formula 1 or a pharmaceutically acceptable salt thereof as an active ingredient. Advantageous Effects

The present disclosure relates to a use of a novel compound for preventing, ameliorating, or treating Parkinson's disease, wherein the novel compound, a PRG-A compound, showed an effect of inhibiting synuclein aggregation in a Parkinson's disease (PD) mouse model. As a result of histological analysis, it was found that treatment of the PRG-A compound blocked loss of dopaminergic neurons. In conclusion, the PRG-A compound may be useful for development as a therapeutic agent for Parkinson's disease.

### Brief Description of Drawings

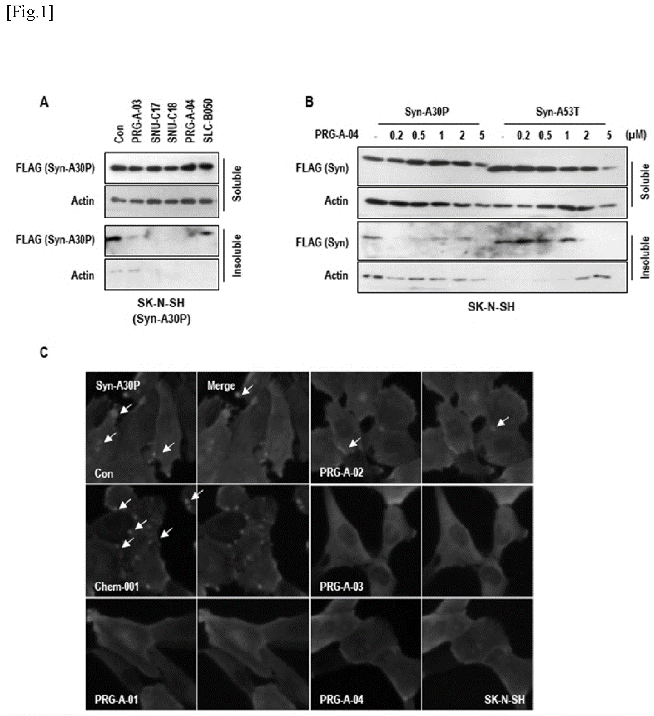

FIG. 1 shows results that a PRG-A compound reduces MT-synuclein aggregation. A. A result showing that a PRG-A compound reduces an insoluble form of MT-synuclein. B. A result showing that PRG-A-04 inhibits MT-synuclein aggregation dose-dependently. C. A result showing that a PRG-A compound eliminates synuclein aggregation in SK-N-SH cells overexpressing Syn-A30P.

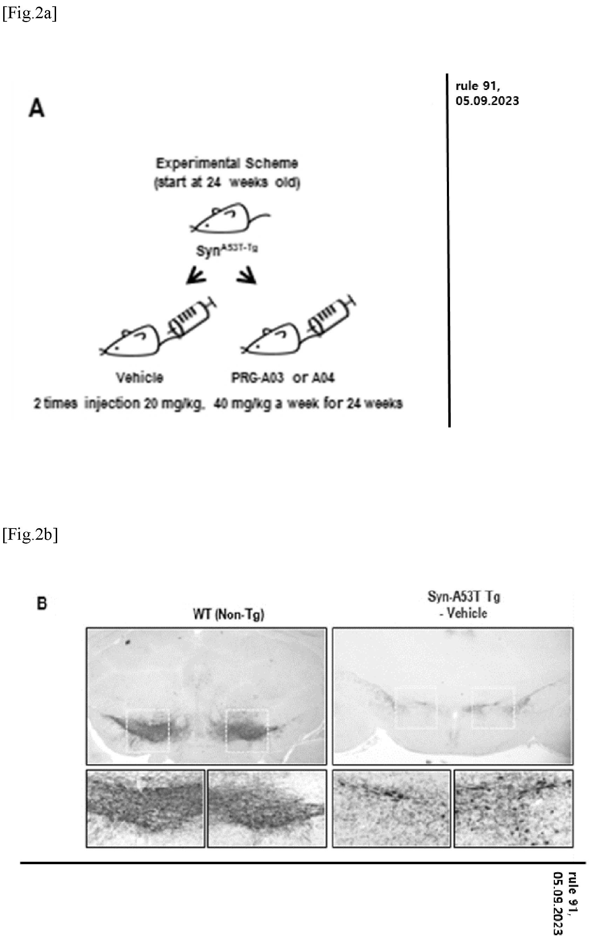

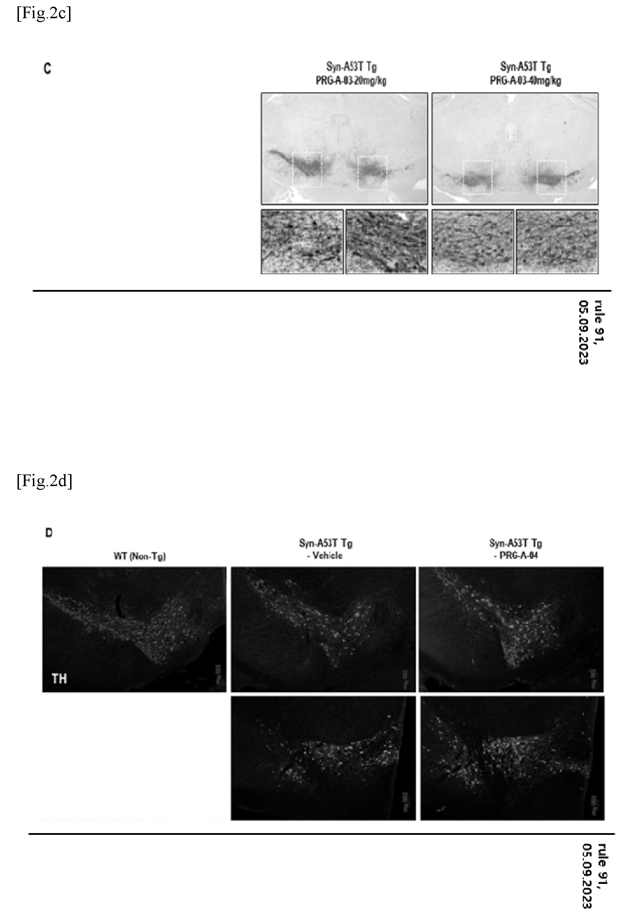

FIG. 2 shows results of identifying an in-vivo effect of a PRG-A compound in a Parkinson's disease (PD) mouse model. A. A schematic diagram of an experiment for administration of a PRG-A compound (PRG-A-03, PRG-A-04). B. A result of measuring dopaminergic neuron loss in a Syn-A53T Tg mouse model. C. A result showing that PRG-A-03 exhibits high expression of synuclein and suppresses phosphorylation of Tau in brain tissue of Syn-A53T Tg. D. A result showing that PRG-A-04 protects against dopaminergic neuron loss in Syn-A53T Tg mice.

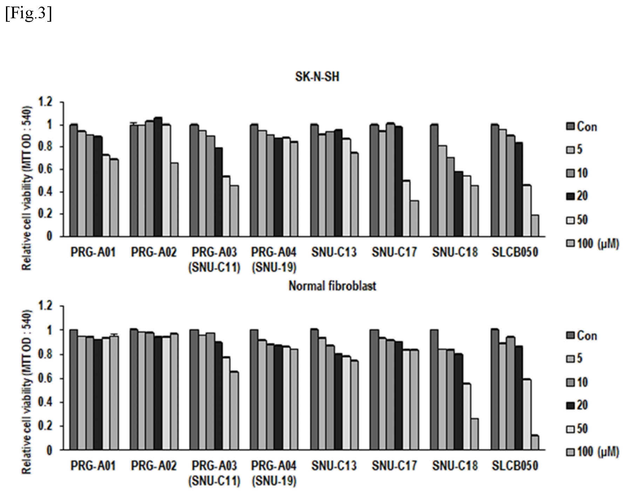

FIG. 3 shows a toxic effect of a PRG-A compound.

### Best Mode for Carrying Out the Invention

Hereinafter, the present disclosure will be described in detail.

The present disclosure provides a pharmaceutical composition for preventing or treating Parkinson's disease, including a compound represented by the following Chemical Formula 1, a hydrate thereof, or a salt thereof.

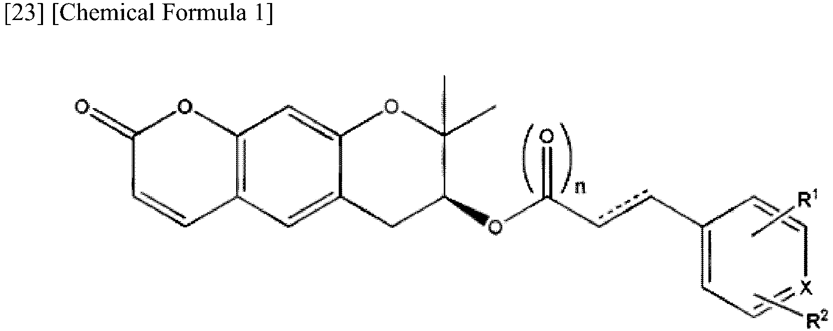

In the Chemical Formula 1,
- - - - - - is a single bond or a double bond,
n is an integer between 0 and 1,
X is CH or N, and
R¹ and R² may be the same or different and each independently selected from hydrogen, (C1~C4)alkyl, (C1~C4)alkoxy, hydroxy, halo, nitro, cyano, or (C1∼C4)alkylcarboxy.

Preferably, in the compound, when - - - - - - is a single bond, n is 1, X is CH, and R¹ and R² may be the same or different and each independently selected from (C1~C4)alkoxy, hydroxy, or (C1~C4)alkylcarboxy, but are not limited thereto.

Preferably, in the compound, when - - - - - - is a double bond, n is an integer between 0 and 1, X is CH or N, and R¹ and R² may be the same or different and each independently selected from hydrogen, (C1~C4)alkoxy, hydroxy, halo, nitro, or (C1~C4)alkylcarboxy, but are not limited thereto.

Preferably, the compound may be a compound represented by the following Chemical Formula 2.

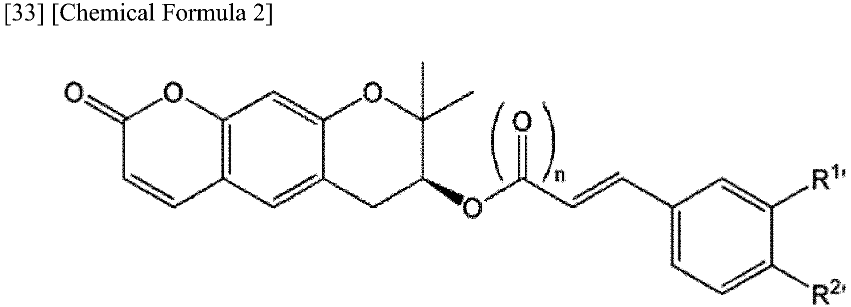

In the Chemical Formula 2,
n is an integer between 0 and 1, and
R¹' and R²' may be the same or different and each independently selected from hydrogen, (C1~C4)alkyl, (C1~C4)alkoxy, hydroxy, halo, nitro, cyano, or (C1∼C4)alkylcarboxy.

The compound represented by the Chemical Formula 1 or Chemical Formula 2 may be named a PRG-A compound.

More preferably, the compound may be selected from the group consisting of, but is not limited to, (S)-8,8-dimethyl-2-oxo-7,8-dihydro-2H,6H-pyrano[3,2-g]chromen-7-yl (E)-3-(4-hydroxy-3-methoxyphenyl)acrylate (PRG-A-01), (S,E)-7-((3-(4-hydroxy-3-methoxyphenyl)allyl)oxy)-8,8-dimethyl-7,8-dihydro-2H,6H-pyrano[3,2-g]chromen-2-one (PRG-A-02), (S)-8,8-dimethyl-2-oxo-7,8-dihydro-2H,6H-pyrano[3,2-g]chromen-7-yl 3-(4-hydroxy-3-methoxyphenyl)propanoate (SNU-C4), (S)-8,8-dimethyl-2-oxo-7,8-dihydro-2H,6H-pyrano[3,2-g]chromen-7-yl (E)-3-(3,4-dimethoxyphenyl)acrylate (SNU-C5), (S)-8,8-dimethyl-2-oxo-7,8-dihydro-2H,6H-pyrano[3,2-g]chromen-7-yl 3-(3,4-dimethoxyphenyl)propanoate (SNU-C7), (S)-8,8-dimethyl-2-oxo-7,8-dihydro-2H,6H-pyrano[3,2-g]chromen-7-yl (E)-3-(pyridin-4-yl)acrylate (SNU-C9), (S)-8,8-dimethyl-2-oxo-7,8-dihydro-2H,6H-pyrano[3,2-g]chromen-7-yl (E)-3-(3-hydroxyphenyl)acrylate (SNU-C10), (S)-8,8-dimethyl-2-oxo-7,8-dihydro-2H,6H-pyrano[3,2-g]chromen-7-yl (E)-3-(4-fluorophenyl)acrylate (PRG-A-03), (S,E)-7-((3-(4-fluorophenyl)allyl)oxy)-8,8-dimethyl-7,8-dihydro-2H,6H-pyrano[3,2-g]chromen-2-one (SNU-C13), (S)-8,8-dimethyl-2-oxo-7,8-dihydro-2H,6H-pyrano[3,2-g]chromen-7-yl (E)-3-(3-acetoxyphenyl)acrylate (SNU-C14), (S)-8,8-dimethyl-2-oxo-7,8-dihydro-2H,6H-pyrano[3,2-g]chromen-7-yl 3-(4-acetoxy-3-methoxyphenyl)propanoate (SNU-C15), (S)-8,8-dimethyl-2-oxo-7,8-dihydro-2H,6H-pyrano[3,2-g]chromen-7-yl (E)-3-(4-acetoxy-3-methoxyphenyl)acrylate (SNU-C17), (S)-8,8-dimethyl-2-oxo-7,8-dihydro-2H,6H-pyrano[3,2-g]chromen-7-yl (E)-3-(3,4-difluorophenyl)acrylate (SNU-C18), and (S,E)-7-((3-(3-methoxy-4-nitrophenyl)allyl)oxy)-8,8-dimethyl-7,8-dihydro-2H,6H-pyrano[3,2-g]chromen-2-one (PRG-A-04).

More preferably, the pharmaceutical composition may inhibit aggregation of synuclein and loss of dopaminergic neurons.

The pharmaceutical composition of the present disclosure may be prepared using pharmaceutically suitable and physiologically acceptable auxiliary agents in addition to the active ingredient, and the auxiliary agents may include solubilizers such as excipients, disintegrants, sweeteners, binders, coating agents, swelling agents, lubricants, glidants, or flavoring agents. The pharmaceutical composition of the present disclosure may be preferably formulated as a pharmaceutical composition by including one or more types of pharmaceutically acceptable carriers in addition to the active ingredient for administration. In a composition formulated as a liquid solution, acceptable pharmaceutical carriers may be those that are sterile and biocompatible and used by mixing saline solution, sterile water, Ringer's solution, buffered saline, albumin injection solution, dextrose solution, maltodextrin solution, glycerol, ethanol, and mixtures of one or more of these components, and other conventional additives, such as antioxidants, buffers, and bacteriostatic agents, may be added as needed. In addition, diluents, dispersants, surfactants, binders, and lubricants may be additionally added to formulate as injectable formulations such as aqueous solutions, suspensions, and emulsions, pills, capsules, granules, or tablets.

The form of the pharmaceutical formulation of the pharmaceutical composition of the present disclosure may be granules, powders, coated tablets, tablets, capsules, suppositories, syrups, juices, suspensions, emulsions, drops or injectable liquids, and sustained-release formulations of active compounds. The pharmaceutical composition of the present disclosure may be administered in a conventional manner via intravenous, intraarterial, intraperitoneal, intramuscular, intraarterial, intraperitoneal, intrasternal, transdermal, intranasal, inhalation, topical, rectal, oral, intraocular, or intradermal routes. The effective amount of the active ingredient of the pharmaceutical composition of the present disclosure refers to an amount required for the prevention or treatment of a disease. Therefore, it may be adjusted according to various factors including the type of disease, severity of the disease, type and content of the active ingredient and other ingredients contained in the composition, type of formulation, and the patient's age, weight, general health status, sex and diet, time of administration, route of administration and secretion rate of the composition, treatment period, and concurrently used drugs. Although not limited to, in the case of adults, for example, when administered once to several times a day, the composition of the present disclosure may be administered at a dosage of 0.01 ng/kg to 10 g/kg when administered once to several times a day.

In addition, the present disclosure provides a health functional food composition for preventing or ameliorating Parkinson's disease, including a compound represented by the following Chemical Formula 1, a hydrate thereof, or a salt thereof.

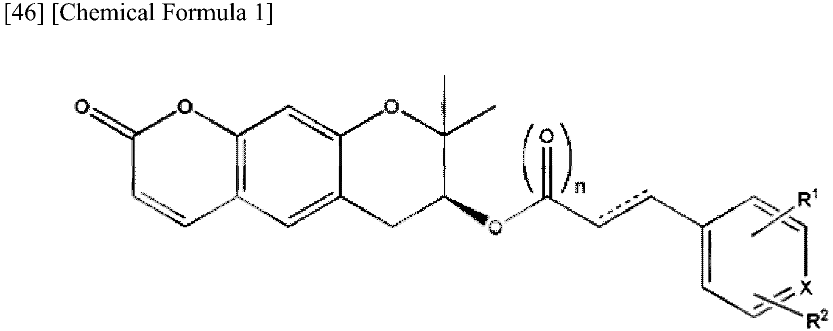

In the Chemical Formula 1,
- - - - - - is a single bond or a double bond,
n is an integer between 0 and 1,
X is CH or N, and
R¹ and R² may be the same or different and each independently selected from hydrogen, (C1~C4)alkyl, (C1~C4)alkoxy, hydroxy, halo, nitro, cyano, or (C1∼C4)alkylcarboxy.

Preferably, in the compound, when - - - - - - is a single bond, n is 1, X is CH, and R¹ and R² may be the same or different and each independently selected from (C1~C4)alkoxy, hydroxy, or (C1~C4)alkylcarboxy, but are not limited thereto.

Preferably, in the compound, when - - - - - - is a double bond, n is an integer between 0 and 1, X is CH or N, and R¹ and R² may be the same or different and each independently selected from hydrogen, (C1~C4)alkoxy, hydroxy, halo, nitro, or (C1~C4)alkylcarboxy, but are not limited thereto.

Preferably, the compound may be a compound represented by the following Chemical Formula 2.

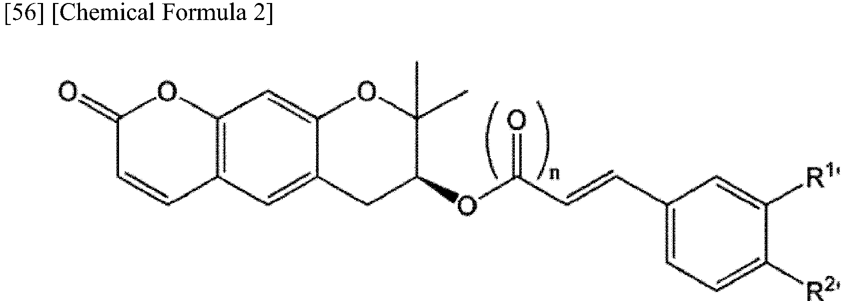

In the Chemical Formula 2,
n is an integer between 0 and 1, and
R¹' and R²' may be the same or different and each independently selected from hydrogen, (C1~C4)alkyl, (C1∼C4)alkoxy, hydroxy, halo, nitro, cyano, or (C1∼C4)alkylcarboxy.

The compound represented by the Chemical Formula 1 or Chemical Formula 2 may be named a PRG-A compound.

More preferably, the compound may be selected from the group consisting of, but is not limited to, (S)-8,8-dimethyl-2-oxo-7,8-dihydro-2H,6H-pyrano[3,2-g]chromen-7-yl (E)-3-(4-hydroxy-3-methoxyphenyl)acrylate (PRG-A-01), (S,E)-7-((3-(4-hydroxy-3-methoxyphenyl)allyl)oxy)-8,8-dimethyl-7,8-dihydro-2H,6H-pyrano[3,2-g]chromen-2-one (PRG-A-02), (S)-8,8-dimethyl-2-oxo-7,8-dihydro-2H,6H-pyrano[3,2-g]chromen-7-yl 3-(4-hydroxy-3-methoxyphenyl)propanoate (SNU-C4), (S)-8,8-dimethyl-2-oxo-7,8-dihydro-2H,6H-pyrano[3,2-g]chromen-7-yl (E)-3-(3,4-dimethoxyphenyl)acrylate (SNU-C5), (S)-8,8-dimethyl-2-oxo-7,8-dihydro-2H,6H-pyrano[3,2-g]chromen-7-yl 3-(3,4-dimethoxyphenyl)propanoate (SNU-C7), (S)-8,8-dimethyl-2-oxo-7,8-dihydro-2H,6H-pyrano[3,2-g]chromen-7-yl (E)-3-(pyridin-4-yl)acrylate (SNU-C9), (S)-8,8-dimethyl-2-oxo-7,8-dihydro-2H,6H-pyrano[3,2-g]chromen-7-yl (E)-3-(3-hydroxyphenyl)acrylate (SNU-C10), (S)-8,8-dimethyl-2-oxo-7,8-dihydro-2H,6H-pyrano[3,2-g]chromen-7-yl (E)-3-(4-fluorophenyl)acrylate (PRG-A-03), (S,E)-7-((3-(4-fluorophenyl)allyl)oxy)-8,8-dimethyl-7,8-dihydro-2H,6H-pyrano[3,2-g]chromen-2-one (SNU-C13), (S)-8,8-dimethyl-2-oxo-7,8-dihydro-2H,6H-pyrano[3,2-g]chromen-7-yl (E)-3-(3-acetoxyphenyl)acrylate (SNU-C14), (S)-8,8-dimethyl-2-oxo-7,8-dihydro-2H,6H-pyrano[3,2-g]chromen-7-yl 3-(4-acetoxy-3-methoxyphenyl)propanoate (SNU-C15), (S)-8,8-dimethyl-2-oxo-7,8-dihydro-2H,6H-pyrano[3,2-g]chromen-7-yl (E)-3-(4-acetoxy-3-methoxyphenyl)acrylate (SNU-C17), (S)-8,8-dimethyl-2-oxo-7,8-dihydro-2H,6H-pyrano[3,2-g]chromen-7-yl (E)-3-(3,4-difluorophenyl)acrylate (SNU-C18), and (S,E)-7-((3-(3-methoxy-4-nitrophenyl)allyl)oxy)-8,8-dimethyl-7,8-dihydro-2H,6H-pyrano[3,2-g]chromen-2-one (PRG-A-04).

The health functional food composition of the present disclosure may further include one or more additives selected from the group consisting of organic acid, phosphate, antioxidant, lactose casein, dextrin, glucose, sugar, and sorbitol. The organic acid may be, but is not limited to, citric acid, fumaric acid, adipic acid, lactic acid, or malic acid, the phosphate may be, but is not limited to, sodium phosphate, potassium phosphate, acid pyrophosphate or polyphosphate, and the antioxidant may be, but is not limited to, a natural antioxidant such as polyphenols, catechins, alpha-tocopherol, rosemary extract, licorice extract, chitosan, tannic acid, or phytic acid.

In another specific example of the present disclosure, the health functional food may contain, in addition to the active ingredient, various nutrients, vitamins, minerals (electrolytes), flavoring agents such as synthetic flavoring agents and natural flavoring agents, coloring agents and thickening agents (cheese, chocolate, etc.), pectic acid and salts thereof, alginic acid and salts thereof, organic acids, protective colloid thickeners, pH regulators, stabilizers, preservatives, glycerin, alcohol, and carbonating agents used in carbonated beverages. Additionally, a food composition according to one embodiment of the present disclosure may contain fruit pulp for producing natural fruit juice, fruit juice beverage, and vegetable beverage.

According to one embodiment of the present disclosure, the formulation of the health functional food may be in the form of a solid, powder, granule, tablet, capsule, liquid, or beverage, but is not limited thereto.

In addition, the health functional food may be used in the manufacture of foods such as, but not limited to, confectionery, sugar, ice cream products, dairy products, meat products, fish meat products, tofu or jelly, edible oils, noodles, tea, beverages, special nutritional foods, health supplements, seasoned foods, ice, ginseng products, kimchi pickled foods, dried foods, fruits, vegetables, dried fruits or vegetables, cut products, fruit juice, vegetable juice, mixed juices thereof, chips, noodles, processed livestock foods, processed fishery foods, processed dairy foods, fermented dairy foods, legume foods, grain foods, microbial fermented foods, confectionery and bakery products, seasonings, processed meat products, acidic beverages, licorice, and herbs.

### Modes for Carrying Out the Invention

Hereinafter, in order to help understand the present disclosure, examples will be described in detail. However, the following examples are only intended to illustrate the content of the present disclosure, and the scope of the present disclosure is not limited to the following examples. The examples of the present disclosure are provided to more completely describe the present disclosure to those of ordinary skill in the art.

### <Experimental Example>

The following experimental examples are intended to provide experimental examples that are commonly applied to each embodiment according to the present disclosure.

### 1. Preparation of compounds and mice

### 1) Compound

The chemical structures and NMR data of compounds used in the present disclosure are as follows.

### (S)-8,8-dimethyl-2-oxo-7,8-dihydro-2H,6H-pyrano[3,2-g]chromen-7-yl (E)-3-(4-hydroxy-3-methoxyphenyl)acrylate (PRG-A-01)

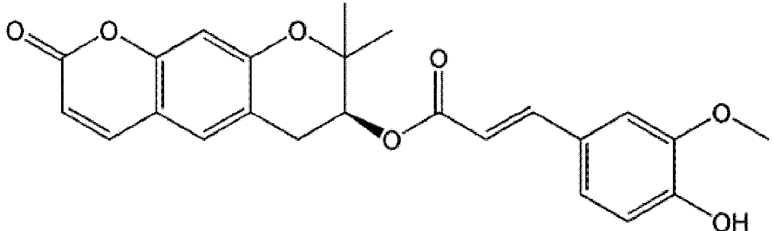

¹H NMR (acetone-d₆, 400MHz)
δ ppm 7.843(d, J=9.6Hz, 1H), 7.616(d, J=16.0Hz, 1H), 7.424(s, 1H), 7.357(s, 1H), 7.123(dd, J=2.0, 8.0Hz, 1H), 6.851(d, J=8.4Hz, 1H), 6.740(s, 1H), 6.387(d, J=15.6Hz, 1H), 6.198(d, J=9.6Hz, 1H), 5.221(t, J=4.6Hz, 1H), 3.895(s, 3H), 3.321(dd, J=4.6, 17.2Hz, 1H), 2.963(dd, J=4.4, 17.6Hz, 1H), 1.421(s, 3H), 1.413(s, 3H); MS(m/z) 423 (M+H)⁺

### (S,E)-7-((3-(4-hydroxy-3-methoxyphenyl)allyl)oxy)-8,8-dimethyl-7,8-dihydro-2H,6H-pyrano[3,2-g]chromen-2-one (PRG-A-02)

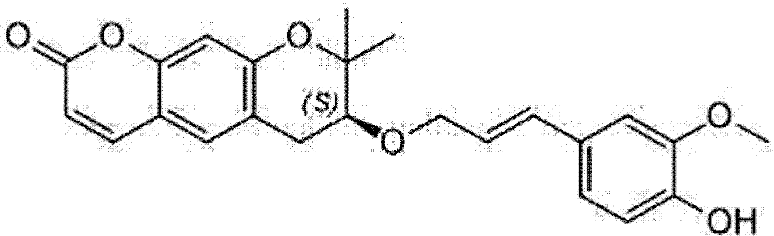

¹H NMR: EW15731-164-P1D4 (400 MHz, CDCl₃)
δ 7.58 (d, J = 12 Hz, 1H), 7.16 (s, 1H), 6.90 - 6.86 (m, 3H), 6.78 (s, 1H), 6.51 (d, J =16 Hz, 1H), 6.22 (d, J = 8.0 Hz, 1H), 6.14 - 6.10 (m, 1H), 5.65 (s, 1H), 4.32 - 4.30 (m, 1H), 4.22- 4.20 (m, 1H), 3.91 (s, 3H), 3.61 - 3.58 (m, 1H), 3.11 - 3.05 (m, 1H), 3.89 - 3.87 (m, 1H), 1.43 (s, 3H), 1.36 (s, 3H).

### (S)-8,8-dimethyl-2-oxo-7,8-dihydro-2H,6H-pyrano[3,2-g]chromen-7-yl (E)-3-(4-fluorophenyl)acrylate (PRG-A-03)

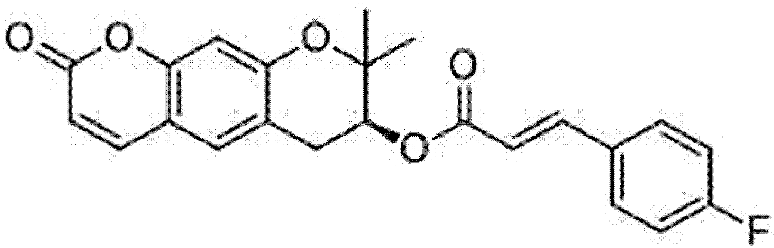

¹H NMR (CD₂Cl₂, 400MHz): δ ppm 7.66 - 7.55 (m, 2H), 7.55 - 7.46 (m, 2H), 7.18 (s, 1H), 7.11 - 7.01 (m, 2H), 6.76 (s, 1H), 6.35 (d, J = 16.0 Hz, 1H), 6.16 (d, J = 9.5 Hz, 1H), 5.17 (app.t, J = 4.7 Hz, 1H), 3.23 (ddd, J = 17.3, 4.8, 1.2 Hz, 1H), 2.92 (dd, J = 17.3, 4.7 Hz, 1H), 1.40 (s, 3H), 1.36 (s, 3H).

### (S,E)-7-((3-(3-methoxy-4-nitrophenyl)allyl)oxy)-8,8-dimethyl-7,8-dihydro-2H,6H-pyrano[3,2-g]chromen-2-one (PRG-A-04)

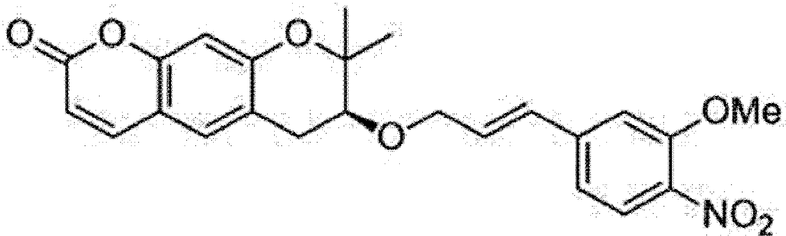

¹H NMR (CDCl₃, 400MHz): δ ppm 7.85 (d, J = 8.4 Hz, 1H), 7.57 (d, J = 9.5 Hz, 1H), 7.16 (s, 1H), 7.05 - 6.97 (m, 2H), 6.78 (s, 1H), 6.60 (app.dt, J = 15.9, 1.7 Hz, 1H), 6.40 (app.dt, J = 15.9, 5.5 Hz, 1H), 6.22 (d, J = 9.4 Hz, 1H), 4.39 (ddd, J = 13.6, 5.4, 1.7 Hz, 1H), 4.24 (ddd, J = 13.4, 5.5, 1.6 Hz, 1H), 3.97 (s, 3H), 3.59 (dd, J = 7.1, 4.9 Hz, 1H), 3.11 (dd, J = 16.7, 4.9 Hz, 1H), 2.87 (dd, J = 16.7, 7.2 Hz, 1H), 1.43 (s, 3H), 1.38 (s, 3H).

### (S,E)-7-((3-(4-fluorophenyl)allyl)oxy)-8,8-dimethyl-7,8-dihydro-2H,6H-pyrano[3,2-g]chromen-2-one (SNU-C13)

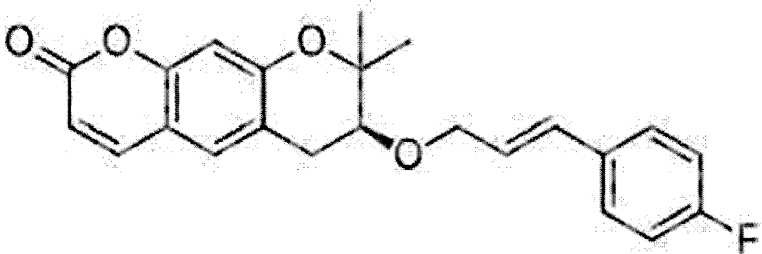

¹H NMR (CDCl₃, 400MHz): δ ppm 7.57 (d, J = 9.5 Hz, 1H), 7.38 - 7.29 (m, 2H), 7.16 (s, 1H), 7.06 - 6.93 (m, 2H), 6.77 (s, 1H), 6.55 (d, J = 15.8 Hz, 1H), 6.25 - 6.13 (m, 2H), 4.33 (ddd, J = 12.8, 5.8, 1.5 Hz, 1H), 4.19 (ddd, J = 12.8, 6.3, 1.4 Hz, 1H), 3.58 (dd, J = 7.4, 5.0 Hz, 1H), 3.08 (dd, J = 16.4, 5.0 Hz, 1H), 2.85 (ddd, J = 16.4, 7.4, 1.1 Hz, 1H), 1.42 (s, 3H), 1.35 (s, 3H).

### (S)-8,8-dimethyl-2-oxo-7,8-dihydro-2H,6H-pyrano[3,2-g]chromen-7-yl (E)-3-(4-acetoxy-3-methoxyphenyl)acrylate (SNU-C17)

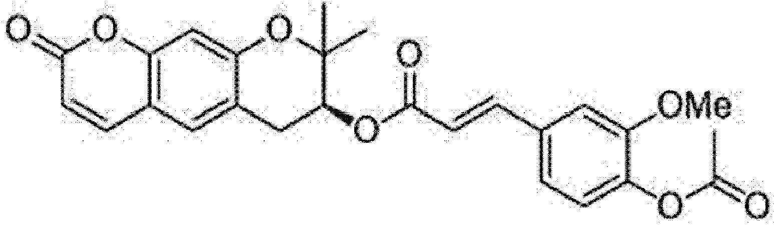

¹H NMR (CD₂Cl₂, 600MHz): δ ppm 7.67 - 7.56 (m, 2H), 7.21 (s, 1H), 7.14 - 7.09 (m, 2H), 7.03 (d, J = 8.0 Hz, 1H), 6.78 (s, 1H), 6.41 (d, J = 16.0 Hz, 1H), 6.19 (d, J = 9.5 Hz, 1H), 5.20 (app.t, J = 4.7 Hz, 1H), 3.82 (s, 3H), 3.26 (ddd, J = 17.3, 4.8, 1.2 Hz, 1H), 2.95 (dd, J = 17.3, 4.5 Hz, 1H), 2.27 (s, 3H), 1.43 (s, 3H), 1.38 (s, 3H).

### (S)-8,8-dimethyl-2-oxo-7,8-dihydro-2H,6H-pyrano[3,2-g]chromen-7-yl (E)-3-(3,4-difluorophenyl)acrylate (SNU-C18)

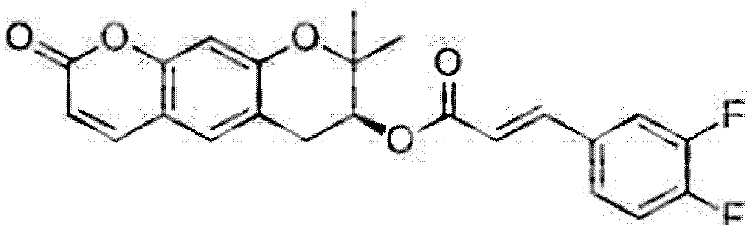

¹H NMR (CDCl₃, 400MHz): δ ppm 7.62 - 7.53 (m, 2H), 7.32 (ddd, J = 11.1, 7.6, 2.2 Hz, 1H), 7.24 - 7.11 (m, 3H), 6.83 (s, 1H), 6.33 (d, J = 16.0, 1H), 6.24 (d, J = 9.4 Hz, 1H), 5.19 (app.t, J = 4.7 Hz, 1H), 3.25 (ddd, J = 17.4, 4.8, 1.1 Hz, 1H), 2.93 (dd, J = 17.4, 4.5 Hz, 1H), 1.43 (s, 3H), 1.39 (s, 3H).

### (7S)-(+)-3-(2-Furanyl)-acrylic acid, 8,8-dimethyl-2-oxo-6,7- dihydro-2H,8H-pyrano[3,2-g]chromen-7-yl-ester (SLC-B050)

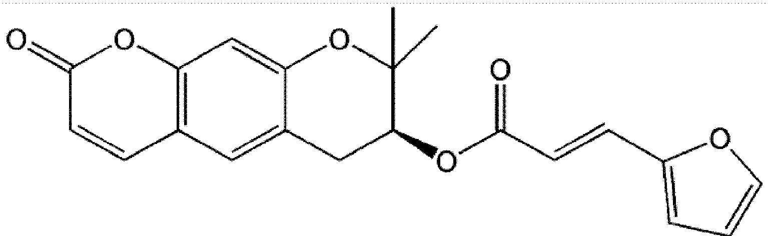

¹H NMR(400 MHz, CDCl₃): δH 7.64(1H, s, H-6'), 7.58(1H, d, J = 9.6Hz, H-4), 7.56(1H, d, J = 16.0Hz, H-3'), 7.41(1H, d, J = 1.6Hz, H-7'), 7.17(1H, s, H-5), 6.82(1H, s, H-10), 6.55(1H, d, J = 1.6Hz, H-8'), 6.23(1H, d, J = 9.6Hz, H-3), 6.13(1H, d, J = 16.0Hz, H-2'), 5.17(1H, t, J = 4.4Hz, H-7), 3.23(1H, dd, J = 4.4, 17.6Hz, H-6a), 2.92(1H, dd, J = 4.4, 17.6Hz, H-6b), 1.42(3H, s, CH3-8), 1.38(3H, s, CH3-8).

### 2) Mice

Experiments were conducted in approved facility of Association for Assessment and Accreditation of Laboratory Animal Care in accordance with animal policies approved by Pusan National University. B6.Cg-Tg (Prnp-SNCAA53T)/J mice were obtained from Jackson Laboratory (Stock No: 006823). All mice were maintained under temperature- and light-controlled conditions (20-23°C, 12 h-12 h light/dark cycle) and provided with autoclaved food and water.

### 2. In vivo drug treatment and histological analysis

24 week-old Syn^{A53T-Tg} mice were administered vehicle (DMSO), PRG-A-03, and PRG-A-04 intraperitoneally twice a week for 24 to 26 weeks. Control mice were treated under the same conditions. Mice were sacrificed at 48-52 weeks of age for histological analysis. After cardiac perfusion with 4% paraformaldehyde, the brains were dissected and embedded in paraffin blocks according to standard tissue processing procedures. Embedded tissue (cervical and lumbar regions of the spinal cord) was cut into 5 µm sections using a tissue sectioner (Leica microtome) and transferred to adhesive-coated slides (Marienfeld laboratory glassware, Germany). For histological pathologic analysis of Syn^{A53T-Tg} mice, immunohistochemistry (IHC) using a dopaminergic neuronal marker (Tyrosine Hydroxylase) was performed after deparaffinization and rehydration. To monitor dopaminergic neurons by immunofluorescence (IF), dissected brains were embedded in agarose holders and sectioned with a vibratome. Thinly sliced tissues were treated with a blocking solution (normal donkey serum diluted 1:500 in 0.1% Triton X-100 PBS) for 1 h. Cells were incubated with anti-TH antibody (diluted 1:500) overnight at 4°C. Finally, cells were incubated with FITC-conjugated secondary antibody at 4°C for 2 h. Cells were washed three times with PBS, and coverslips were mounted using mounting solution (H-5501, Vector Laboratories), followed by analysis by fluorescence microscopy (Zeiss).

### 3. Cell culture and reagents

SK-N-SH cells were purchased from the Korean Cell Line Bank (KCLB, Seoul, South Korea) and maintained in MEM medium containing 10% fetal bovine serum, 1% antibiotics, 25 mM HEPES, and 300 mg/L L-Glu. Human fibroblasts (9-year-old female) were obtained from Coridll Cell Repositories (New Jersey, USA) and maintained in EMEM containing 15% FBS and 2 mM glutamine, along with 26 mM HEPES without antibiotics.

### 4. Western blot analysis

For SDS-PAGE, proteins were extracted from cells using RIPA buffer (50 mM Tris-Cl, pH 7.5, 150 mM NaCl, 1% NP-40, 0.1% SDS, and 10% sodium deoxycholate). Samples were separated by SDS-PAGE and transferred to a PVDF membranes. The blotted membrane was blocked with 3% skim milk containing TBST buffer for 1 h and incubated with specific antibodies. Reacted antibodies were detected by ECL and X-ray film exposure. The antibody used in this experiment was pan-Syn (GTX112799), purchased from Genetex (California, USA). Actin antibody (66009-1-lg) and α-tubulin antibody (66240-1-lg) were obtained from Proteintech (Rosemont, IL, USA). Anti-FLAG (Sigma; F3165) from Sigma Aldrich (St, Louis, MO, USA) was used as a secondary antibody, and HRP-conjugated goat anti-mouse, goat anti-rabbit, and mouse anti-goat antibodies (Pierce, Thermo Fisher Scientific, Inc., Rockford, IL, USA) were used. FLAG-Syn-WT and MT vectors were transfected into SK-N-SH cells for 24 h. After incubation, cells were harvested with TNN buffer (50 mM Tris-Cl, pH 7.5, 150 mM NaCl, 0.3% NP-40) and centrifuged at 14,000 rpm for 30 min to separate the pellet (insoluble) and supernatant (soluble). Input represents whole cell lysate (WCL) harvested with RIPA buffer. Actin was used as a loading control.

### 5. Immunofluorescence staining

Cells on coverslips were washed with PBS, fixed with 4% paraformaldehyde (PFA) for 30 min at room temperature, and then permeabilized in 0.1% Triton X-100/PBS for 10 min. After treating cells with a blocking solution (3% anti-human antibody diluted 1:500 in PBS) for 1 h, cells were incubated with anti-FLAG (diluted 1:400) overnight at 4°C. Finally, cells were incubated with FITC- and rhodamine-conjugated secondary antibodies for 6 h at 4°C. Nuclei were stained with 4,6-diamidino-2-phenylindole (DAPI), and endoplasmic reticulum (ER) was stained with ER-Tracker Red dye for 10 min. Cells were washed three times with PBS, and then coverslips were mounted with mounting solution (H-5501; Vector Laboratories, Burlingame, CA, USA), followed by analysis with fluorescence microscopy (Zeiss).

### 6. Transfection of vectors

FLAG-Syn (WT, A30P, and A53T) vectors were purchased from Dr. Seol (Wonkwang University Korea). Transfection was performed using Jet-PEI reagent (JetPEI; Polyplus transfection, New York, NY, USA) according to the manufacturer's protocol. Briefly, the vector was mixed with JetPEI reagent in 150 mM NaCl buffer, and the mixture was incubated for 15 minutes. The mixture was added to cells in serum-free medium for 4 h. After incubation, the cells were replaced with culture medium supplemented with 10% FBS.

### 7. Measurement of cell viability

To investigate cell viability, cells were treated with chemicals (PRG-A compound) for 48 h. Then, the cells were incubated with 0.5 mg/ml 3-(4,5-dimethylthiazol-2-yl)-2,5-diphenyltetrazolium bromide (MTT) solution (475989; Merck, Darmstadt, Germany) at 37°C for 4 h. After removing the excess solution and washing with PBS, the precipitated substances were dissolved in 200 µl of DMSO and quantified by measuring the absorbance at 540 nm.

### <Example 1>

To identify the effect of the PRG-A compound on alpha-synuclein aggregation, the inventors transfected the FLAG-Syn-A30P vector into SK-N-SH cells for 24 h. After incubation, cells were treated with compounds (5 µM) for 24 h. As a result, a large portion of the ectopically expressed mutant alpha-synuclein (Syn-A30P) was recovered as an insoluble fraction (FIG. 1A). However, treatment with the PRG-A compound clearly reduced synuclein aggregation (FIG. 1A). Additionally, after transfection with the indicator vector (MT-Syn) for 24 h in SK-N-SH cells, PRG-A-04 was treated for 24 h. Cells were then harvested with TNN buffer (50 mM Tris-Cl, pH 7.5, 150 mM NaCl, 0.3% NP-40) and centrifuged at 14,000 rpm for 30 min to separate the pellet (insoluble) and supernatant (soluble). Actin was used as a loading control (FIG. 1B). Similar effects were observed in experiments with another synuclein mutant (Syn-A53T) (FIG. 1B), where PRG-A-04 was able to reduce insoluble synuclein in a dose-dependent manner. To observe this, immunofluorescence staining was performed with Syn-Ab. Cells were transfected with Syn-A53T vector for 24 h. After culture, cells were treated with PRG-A compound (5 µM) for 24 h and fixed with 4% PFA. Aggregated synuclein (white arrows) was dissolved by treatment with PRG-A compound (FIG. 1C). Expression of synuclein was observed under a fluorescence microscope with a pan-Syn antibody (white arrow; high intensity of Syn). Chem-001 (decursinol) showed marginal effects on inhibition of synuclein aggregation.

### <Example 2>

To investigate in vivo effects of PRG-A compounds, 24-week-old mice were intraperitoneally injected with 20 mg/kg or 40 mg/kg of PRG-A compounds (PRG-A-03, PRG-A-04) for 24 to 26 weeks, and the mice were sacrificed for histological analysis, and brain tissues were dissected at 48 to 49 weeks of age (FIG. 2A). Dopaminergic neurons in the substantia nigra (SN) were observed using tyrosine hydroxylase (TH) staining (FIG. 2B). Dopaminergic neuron markers (Tyrosine Hydroxylase, TH) are maintained in PRG-A-03-injected mice. Representative images are shown with 10x magnification and 20x insert images (yellow boxes) (FIG. 2B). Compound treatment may block loss of dopaminergic neurons (FIG. 2B). Additionally, expression of mutant Syn was reduced in brain tissue (FIG. 2C). Additionally, the dissected brain was embedded in an agarose holder and sectioned with a vibratome. Thinly sliced tissues were stained with TH antibody (FIG. 2D). As a result, similar results were derived by treating PRG-A-04, which prevents the loss of dopaminergic neurons in the SN by IF staining using TH (FIG. 2D). These results indicate that the PRG-A compound not only blocks synuclein (Syn) aggregation but also protects against neuronal loss in Parkinson's disease (PD).

### <Example 3>

Lastly, to determine the toxicity of PRG-A compounds, normal human fibroblasts and neuroblastoma cells (SK-N-SH) were cultured with each compound for 48 h, and cell viability was measured via the MTT assay. The effect on survival rates of neuronal cells and normal fibroblasts was identified (FIG. 3). The PRG-A-01, PRG-A-02, and PRG-A-04 compounds did not exhibit cytotoxic effects in normal human fibroblasts. The PRG-A-03 compound showed a low impact on cell viability at high doses (50 and 100 µM). In summary, the novel compound did not show toxicity up to 50 µM in normal cells, so the side effects of the new compound are not expected to be serious.

In conclusion, the novel compound may be used to treat Parkinson's disease (PD) without serious side effects.

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

## Claims

1. A pharmaceutical composition for preventing or treating Parkinson's disease, comprising a compound represented by the following Chemical Formula 1, a hydrate thereof, or a salt thereof: 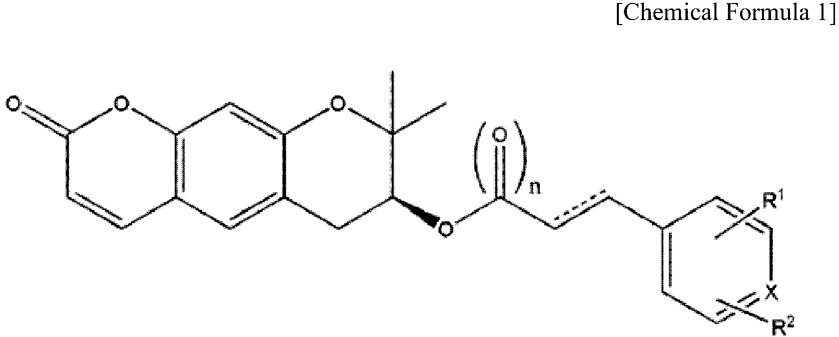 wherein, in the Chemical Formula 1,
- - - - - - is a single bond or a double bond,
n is an integer between 0 and 1,
X is CH or N, and
R¹ and R² are the same or different and each independently selected from hydrogen, (C1~C4)alkyl, (C1~C4)alkoxy, hydroxy, halo, nitro, cyano, or (C1~C4)alkylcarboxy.

2. The pharmaceutical composition of claim 1, wherein, in the compound, when - - - - - - is a single bond, n is 1, X is CH, and R¹ and R² are the same or different and each independently selected from (C1~C4)alkoxy, hydroxy, or (C1~C4)alkylcarboxy.

3. The pharmaceutical composition of claim 1, wherein, in the compound, when ------ is a double bond, n is an integer between 0 and 1, X is CH or N, and R¹ and R² are the same or different and each independently selected from hydrogen, (C1~C4)alkoxy, hydroxy, halo, nitro, or (C1~C4)alkylcarboxy.

4. The pharmaceutical composition of claim 1, wherein the compound is selected from the group consisting of (S)-8,8-dimethyl-2-oxo-7,8-dihydro-2H,6H-pyrano[3,2-g]chromen-7-yl (E)-3-(4-hydroxy-3-methoxyphenyl)acrylate, (S,E)-7-((3-(4-hydroxy-3-methoxyphenyl)allyl)oxy)-8,8-dimethyl-7,8-dihydro-2H,6H-pyrano[3,2-g]chromen-2-one, (S)-8,8-dimethyl-2-oxo-7,8-dihydro-2H,6H-pyrano[3,2-g]chromen-7-yl 3-(4-hydroxy-3-methoxyphenyl)propanoate, (S)-8,8-dimethyl-2-oxo-7,8-dihydro-2H,6H-pyrano[3,2-g]chromen-7-yl (E)-3-(3,4-dimethoxyphenyl)acrylate, (S)-8,8-dimethyl-2-oxo-7,8-dihydro-2H,6H-pyrano[3,2-g]chromen-7-yl 3-(3,4-dimethoxyphenyl)propanoate, (S)-8,8-dimethyl-2-oxo-7,8-dihydro-2H,6H-pyrano[3,2-g]chromen-7-yl (E)-3-(pyridin-4-yl)acrylate, (S)-8,8-dimethyl-2-oxo-7,8-dihydro-2H,6H-pyrano[3,2-g]chromen-7-yl (E)-3-(3-hydroxyphenyl)acrylate, (S)-8,8-dimethyl-2-oxo-7,8-dihydro-2H,6H-pyrano[3,2-g]chromen-7-yl (E)-3-(4-fluorophenyl)acrylate, (S,E)-7-((3-(4-fluorophenyl)allyl)oxy)-8,8-dimethyl-7,8-dihydro-2H,6H-pyrano[3,2-g]chromen-2-one, (S)-8,8-dimethyl-2-oxo-7,8-dihydro-2H,6H-pyrano[3,2-g]chromen-7-yl (E)-3-(3-acetoxyphenyl)acrylate, (S)-8,8-dimethyl-2-oxo-7,8-dihydro-2H,6H-pyrano[3,2-g]chromen-7-yl 3-(4-acetoxy-3-methoxyphenyl)propanoate, (S)-8,8-dimethyl-2-oxo-7,8-dihydro-2H,6H-pyrano[3,2-g]chromen-7-yl (E)-3-(4-acetoxy-3-methoxyphenyl)acrylate, (S)-8,8-dimethyl-2-oxo-7,8-dihydro-2H,6H-pyrano[3,2-g]chromen-7-yl (E)-3-(3,4-difluorophenyl)acrylate, and (S,E)-7-((3-(3-methoxy-4-nitrophenyl)allyl)oxy)-8,8-dimethyl-7,8-dihydro-2H,6H-pyrano[3,2-g]chromen-2-one.

5. The pharmaceutical composition of claim 1, wherein the compound is represented by the following Chemical Formula 2: 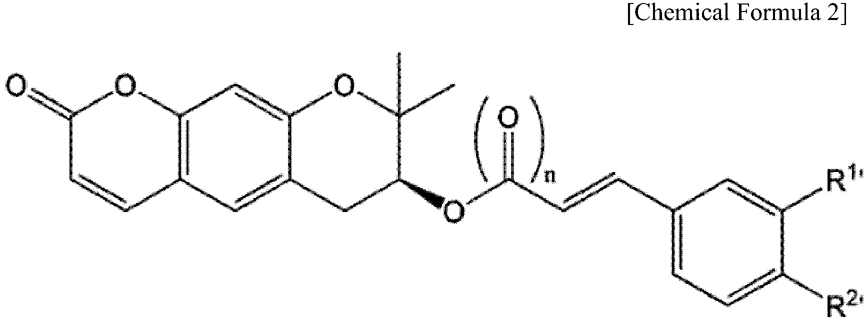 wherein, in the Chemical Formula 2,
n is an integer between 0 and 1, and
R¹' and R²' are the same or different and each independently selected from hydrogen, (C1~C4)alkyl, (C1~C4)alkoxy, hydroxy, halo, nitro, cyano, or (C1~C4)alkylcarboxy.

6. The pharmaceutical composition of claim 1, wherein the pharmaceutical composition inhibits aggregation of synuclein and loss of dopaminergic neurons.

7. A health functional food composition for preventing or ameliorating Parkinson's disease, comprising a compound represented by the following Chemical Formula 1, a hydrate thereof, or a salt thereof: 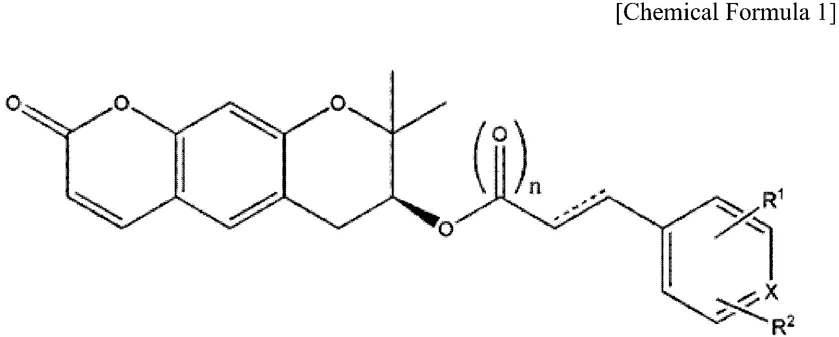 wherein, in the Chemical Formula 1,
- - - - - - is a single bond or a double bond,
n is an integer between 0 and 1,
X is CH or N, and
R¹ and R² are the same or different and each independently selected from hydrogen, (C1~C4)alkyl, (C1~C4)alkoxy, hydroxy, halo, nitro, cyano, or (C1~C4)alkylcarboxy.

8. The health functional food composition of claim 7, wherein, in the compound, when - - - - - - is a single bond, n is 1, X is CH, and R¹ and R² are the same or different and each independently selected from (C1~C4)alkoxy, hydroxy, or (C1~C4)alkylcarboxy.

9. The health functional food composition of claim 7, wherein, in the compound, when - - - - - - is a double bond, n is an integer between 0 and 1, X is CH or N, and R¹ and R² are the same or different and each independently selected from hydrogen, (C1~C4)alkoxy, hydroxy, halo, nitro, or (C1~C4)alkylcarboxy.

10. The health functional food composition of claim 7, wherein the compound is selected from the group consisting of (S)-8,8-dimethyl-2-oxo-7,8-dihydro-2H,6H-pyrano[3,2-g]chromen-7-yl (E)-3-(4-hydroxy-3-methoxyphenyl)acrylate, (S,E)-7-((3-(4-hydroxy-3-methoxyphenyl)allyl)oxy)-8,8-dimethyl-7,8-dihydro-2H,6H-pyrano[3,2-g]chromen-2-one, (S)-8,8-dimethyl-2-oxo-7,8-dihydro-2H,6H-pyrano[3,2-g]chromen-7-yl 3-(4-hydroxy-3-methoxyphenyl)propanoate, (S)-8,8-dimethyl-2-oxo-7,8-dihydro-2H,6H-pyrano[3,2-g]chromen-7-yl (E)-3-(3,4-dimethoxyphenyl)acrylate, (S)-8,8-dimethyl-2-oxo-7,8-dihydro-2H,6H-pyrano[3,2-g]chromen-7-yl 3-(3,4-dimethoxyphenyl)propanoate, (S)-8,8-dimethyl-2-oxo-7,8-dihydro-2H,6H-pyrano[3,2-g]chromen-7-yl (E)-3-(pyridin-4-yl)acrylate, (S)-8,8-dimethyl-2-oxo-7,8-dihydro-2H,6H-pyrano[3,2-g]chromen-7-yl (E)-3-(3-hydroxyphenyl)acrylate, (S)-8,8-dimethyl-2-oxo-7,8-dihydro-2H,6H-pyrano[3,2-g]chromen-7-yl (E)-3-(4-fluorophenyl)acrylate, (S,E)-7-((3-(4-fluorophenyl)allyl)oxy)-8,8-dimethyl-7,8-dihydro-2H,6H-pyrano[3,2-g]chromen-2-one, (S)-8,8-dimethyl-2-oxo-7,8-dihydro-2H,6H-pyrano[3,2-g]chromen-7-yl (E)-3-(3-acetoxyphenyl)acrylate, (S)-8,8-dimethyl-2-oxo-7,8-dihydro-2H,6H-pyrano[3,2-g]chromen-7-yl 3-(4-acetoxy-3-methoxyphenyl)propanoate, (S)-8,8-dimethyl-2-oxo-7,8-dihydro-2H,6H-pyrano[3,2-g]chromen-7-yl (E)-3-(4-acetoxy-3-methoxyphenyl)acrylate, (S)-8,8-dimethyl-2-oxo-7,8-dihydro-2H,6H-pyrano[3,2-g]chromen-7-yl (E)-3-(3,4-difluorophenyl)acrylate, and (S,E)-7-((3-(3-methoxy-4-nitrophenyl)allyl)oxy)-8,8-dimethyl-7,8-dihydro-2H,6H-pyrano[3,2-g]chromen-2-one.

11. The health functional food composition of claim 7, wherein the compound is represented by the following Chemical Formula 2: 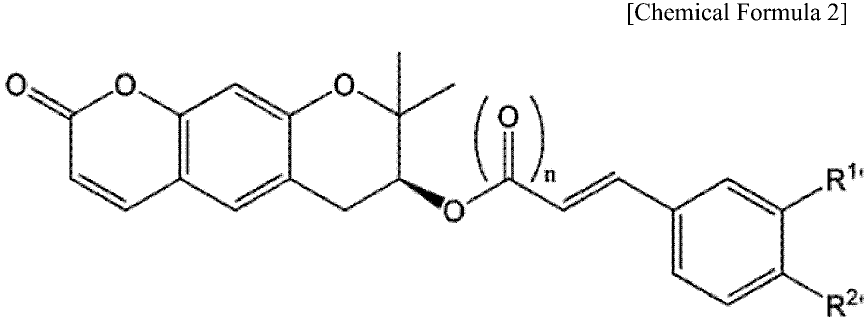 wherein, in the Chemical Formula 2,
n is an integer between 0 and 1, and
R¹' and R²' are the same or different and each independently selected from hydrogen, (C1~C4)alkyl, (C1~C4)alkoxy, hydroxy, halo, nitro, cyano, or (C1~C4)alkylcarboxy.
